# EUROPEAN PATENT SPECIFICATION

(11) **EP 2 155 162 B1**
(45) Date of publication and mention of the grant of the patent: **21.11.2012**
(21) Application number: 08747283.3
(22) Date of filing: 01.05.2008
(51) Int. Cl.: A61K 47/12, A61K 31/715

(54) **COMPOSITIONS FOR REDUCING, AMELIORATING, TREATING, OR PREVENTING CONDITION OF DRY EYE AND METHODS OF MAKING AND USING SAME**
ZUSAMMENSETZUNGEN ZUR REDUZIERUNG, LINDERUNG, BEHANDLUNG ODER VERHÜTUNG VON TROCKENEN AUGEN UND VERFAHREN ZU IHRER HERSTELLUNG UND VERWENDUNG
COMPOSITIONS POUR RÉDUIRE, AMÉLIORER, TRAITER, OU EMPÊCHER L'AFFECTION DE KÉRATOCONJONCTIVITE SÈCHE, ET LEURS PROCÉDÉS DE FABRICATION ET D'UTILISATION

(30) Priority: 04.05.2007 US 916046 P
(43) Date of publication of application: 24.02.2010
(73) Proprietor: Bausch & Lomb Incorporated, Rochester, NY 14604-2701 (US)
(72) Inventor: XIA, Erning, Penfield, NY 14526 (US); DOBIE, Alyce, K., Williamson, NY 114513 (US); KLEIBER, Tammy, J., Rochester, NY 14616 (US)
(74) Representative: Glas, Holger
(86) International application number: PCT/US2008/062141
(87) International publication number: WO 2008/137496

(56) References cited:
- EP-A- 0 861 658
- EP-A- 1 213 018
- WO-A-03/013275
- WO-A-2006/105384
- US-A- 4 960 799
- US-A1- 2007 004 672

## Description

### BACKGROUND

The present invention relates to compositions for reducing, ameliorating, treating, or preventing condition of dry eye, and methods of making and using such compositions. In particular, the present invention relates to compositions for use in reducing, ameliorating, treating, or preventing discomfort of dry eye condition.

Dry eye, also known as keratoconjunctivitis sicca or dyslacrima, is a common ophthalmological disorder affecting millions of people. A patient with dry eye may experience burning, a feeling of dryness, and persistent irritation. In severe cases, dry eye can seriously impair a person's vision and hence handicap the sufferer in activities such as driving. Certain diseases such as Sjogren's disease manifest dry eye symptoms. Also, as people age, the lacrimal glands in the eye may produce less moisture, resulting in eyes that become dry, inflamed, itchy, and gritty.

Although it appears that dry eye may result from a variety of unrelated pathogenic causes, all presentations of the condition share a common feature, namely the breakdown of the precorneal tear film, which breakdown commonly results in dehydration of the exposed outer ocular surface and hence the symptoms described above.

A number of approaches exist for the treatment of dry eye. One common approach has been to supplement the ocular tear film using artificial tears instilled throughout the day. Examples of the tear substitute approach include the use of buffered, isotonic saline solutions and aqueous solutions containing water-soluble polymers that render the solutions more viscous and thus less easily shed by the eye by the washing action of the tear fluid. See, for example, U.S. Patent 5,209,927 to Gressel et al.; U.S. Patent 5,294,607 to Glonek et al.; and U.S. Patent 4,409,205 to Shively;

Although these approaches have met with some success in some cases, significant challenges in the treatment of dry eye nevertheless remain. Problems include the fact that the use of tear substitutes, while temporarily effective, generally requires repeated application over the course of a patient's waking hours, not uncommonly ten or more times over the course of a day. Such an approach is inconvenient to a patient. Although increasing the viscosity of the dry-eye product may extend the product's duration in the eye, increase in viscosity is effective at extending duration only to a limited extent. Viscous ophthalmic drops are sometimes undesirable because they feel sticky in the eye. Further, increases in the duration of the product would be highly desirable.

Alginate, for the purpose of this application is a polysaccharide that comprises monomeric units of β-D-mannuronic acid and α-L-guluronic acid, or salts thereof, or derivatives of such acids or salts.

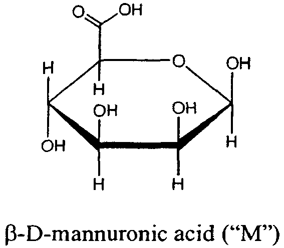

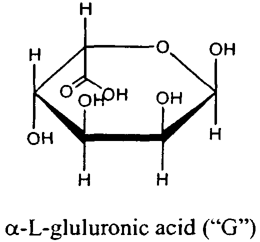

Some alginate polymers are block copolymers with blocks of the guluronic acid (or a salt thereof) monomeric units alternating with blocks of the mannuronic acid (or a salt thereof) monomeric units. Other alginate molecules have alternating single monomeric units of guluronic acid (or a salt thereof) and mannuronic acid (or a salt thereof). The ratio and distribution of the M and G components along with the average molecular weight affect the physical and chemical properties of the copolymer. See A. Haug et al., Acta Chem Scand, Vol. 20, 183-190 (1966). Alginate polymers have viscoelastic rheological properties and other properties that make it suitable for some medical applications. See G. Klock et al., "Biocompatibility of Mannuronic Acid-Rich Alginates," Biomaterials, Vol. 18, No. 10, 707-713 (1997).

The use of alginate as a thickener for topical ophthalmic use is disclosed in U.S. Patent 6,528,465 and U.S. Patent Application Publication 2003/0232089. U.S. Patent 5,776,445 discloses the use of alginate as a drug delivery agent that is topically applied to the eye. Particularly, the amount of guluronic acid in the alginate was taught to exceed 50%.

U.S. Patent Application Publication 2003/0232089 teaches a dry-eye formulation that contains two polymer ingredients including alginate.

Ophthalmic compositions typically include other ingredients that provide additional properties. For example, polyols (e.g., glycerin) are known as demulcents and tonicity adjusting agents in ophthalmic formulations including formulations for the delivery of an active pharmaceutical agent. See; e.g., U.S. Patents 5,075,104 and 5,209,927, which teach the use of a polyol with a cabomer polymer.

In addition, pharmaceutical compositions, including those for ophthalmic applications, very often include an antimicrobial preservative to allow for multiple uses. Some common preservatives that have been used in ophthalmic formulations include benzalkonium chloride, chlorobutanol, alexidine, chlorhexidine, hexamethylene biguanides, quaternary ammonium compounds, and parabens. See; e.g., U.S. Patents 6,833,358; 6,852,311; 6,960,575; and 7,105,473. However, these preservatives can result in some discomfort to sensitive patients, especially those who already suffer from dry eye condition.

US 2007/0004672 A1 relates to a composition for treating dry eye comprising, in one embodiment, alginate and a polyol. Such compositions have been found to alleviate the symptoms of dry eye and remain in the eye for a long period of time.

EP 0 861 658 discloses a preservative for emulsion comprising sorbic acid or a pharmaceutically acceptable salt thereof, and, where necessary, sodium edentate and boric acid. The sorbic acid or a pharmaceutically acceptable salt thereof and emulsions comprising them can impart superior preservation capability to emulsions, such as water in oil type emulsions.

Therefore, in view of the shortcomings of prior-art compositions, there is a continued need to provide improved compositions for the reduction, amelioration, treatment, or prevention of the discomfort resulting from the dry eye condition. It is also desirable to provide such compositions that are gentle to the ocular surface.

### SUMMARY

In general, the present invention provides a composition that is capable of reducing, ameliorating, treating, or preventing discomfort resulting from a condition of dry eye.

In one aspect, the composition has lower risk of introducing unwanted exogenous side effects, such as an unwanted sensation. Alternatively, the composition is gentle to the ocular surface.

The composition of the present invention comprises: (a) alginate; and (b) a combination of at least two organic acids or salts thereof; wherein the composition has a pH in a range from about 5.5 to about 6.5.

In still another aspect, at least one of the two organic acids or salts thereof has a pKa value in the range from about 6 to about 10.

In yet another aspect, each of the two organic acids or a salt thereof has a pKa value in the range from about 4 to about 10, and at least one organic acid or a salt thereof has a pKa value in the range from about 6 to about 10.

In a further aspect, one of the two carboxylic acids or a salt thereof is an aminocarboxylic acid or an iminocarboxylic acid or a salt thereof.

The composition of the present invention further comprises a polyol.

It is further described that the polyol has 2 to 18 (or, alternatively, 2 to 12, or 2 to 10, or 2 to 6, or 2 to 4) carbon atoms.

In a further aspect, the present invention also provides a compound for use in reducing, ameliorating, treating, or preventing a condition of dry eye. The use comprises administering to an eye of a subject suffering from such a condition any one of the compositions herein disclosed.

In still another aspect, such a composition comprises a solution, a dispersion, an emulsion (such as oil-in-water emulsion), a gelable composition, or a gel.

Further is disclosed a method for preparing a pharmaceutical composition. The method comprises combining alginate, at least two organic acids or salts thereof, and a pharmaceutically acceptable carrier to form a mixture having a pH in a range from about 5 to about 7.5; wherein at least one of the organic acid or a salt thereof has a pKa value in a range from about 5 to about 10.

Other features and advantages of the present invention will become apparent from the following detailed description and claims.

### DETAILED DESCRIPTION

In general, the present invention provides a composition that is capable of reducing, ameliorating, treating, or preventing discomfort resulting from a dry eye condition.

In one aspect, the composition has lower risk of introducing unwanted exogenous side effects, such as an unwanted irritating, burning, or stinging sensation. Alternatively, the composition is gentle to the ocular surface.

In another embodiment, the composition has a pH in the range from about 5.5 to about 6.5, or from about 5 to about 6.8, or from about 5.5 to about 6.8.

In yet another aspect, said alginate is present in an amount from about 0.1 to about 0.5, or from about 0.1 to about 0.3 percent by weight of the total composition.

In one embodiment, said alginate comprises alternating homopolymeric blocks, each comprising or consisting of monomeric units of mannuronic acid (or a salt thereof) ("M") or guluronic acid (or a salt thereof) ("G"). In another embodiment, said alginate comprises alternating single units of M and G.

In certain embodiments, said alginate has a molecular weight in a range from about 50 kDa to about 5000 kDa. Alternatively, said alginate has a molecular weight in a range from about 50 kDa to about 2000 kDa (or from about 50 kDa to about 1000 kDa, or from about 50 kDa to about 700 kDa, from about 50 kDa to about 500 kDa, or from about 50 kDa to about 100 kDa, or from about 100 kDa to about 2000 kDa, or from about 100 kDa to about 1000 kDa, or from about 100 kDa to about 500 kDa, or from about 500 kDa to about 2000 kDa, or from about 500 kDa to about 1000 kDa). Suitable alginates are known under the trade name Protanal, available from FMC BioPolymer, Philadelphia, Pennsylvania.

In one preferred embodiment, the molecular weight is about 200-300 kDa.

The proportion ofG monomeric units in an alginate molecule suitable for a composition of the present invention can be in the range from about 10 to about 90 percent of the total number of monomeric units of the alginate molecule. Alternatively, such proportion can be in the range from about 20 to about 75 (or from 30 to about 60, or from about 25 to about 50, or from about 20 to about 50, or from about 10 to about 30) percent of the total number of monomeric units of the alginate molecule. In one embodiment, the such proportion is about 35-45 percent.

In still another aspect, at least one of said at least two organic acids or a salt thereof included in the composition has a pKa value in the range from about 5 to about 10 (or, alternatively, from about 6 to about 8.5).

In yet another aspect, each of said at least two organic acids or salts thereof has a pKa value in the range from about 4 to about 10, and at least one organic acid or a salt thereof has a pKa value in the range from about 5 to about 10 (or alternatively, from about 5.5 to about 9, or from about 6 to about 10, or from about 6 to about 9, or from about 6 to about 8.5). In one embodiment, at least one of the two organic acids has a pKa greater than the pH of the composition. At least one of the two organic acids has a pKa that is at least one half unit greater than the pH of the composition. In still another embodiment, at least one of the two organic acids has a pKa that is at least 1 unit greater than the pH of the composition. It should be noted that a polycarboxylic acid has several pKa values, one or more of said pKa values need be greater than the pH of the composition. Preferably, a plurality of the pKa values of the polycarboxylic acid is greater than the pH of the composition. More preferably, a majority of the pKa values of the polycarboxylic acid is greater than the pH of the composition. Most preferably, all of the pKa values of the polycarboxylic acid are greater than the pH of the composition.

In a further aspect, the first of said two carboxylic acids is an aminocarboxylic acid or an iminocarboxylic acid.

Non-limiting examples of aminocarboxylic acids include ethylenediaminetetraacetic acid ("EDTA", pKa of 1.70, 2.60, 6.30, and 10.60), hexamethylenediaminetetraacetic acid ("HMDTA"), N-(2-hydroxyethyl)ethylenediamine-N,N',N'-triacetic acid ("HEEDTA" or HEDTA"), hydroxymethylethylenediaminetriacetic acid ("HMEDTA"), 1,3-diamino-2-propanol-N,N,N',N'-tetracetic acid, 1,3-diamino-2-propane-N,N,N',N'-tetracetic acid, ethylene glycol-bis(2-aminoethylether)-N,N,N',N'-tetraacetic acid, ethylenediamine-N,N-diacetic acid ("EDDA", pKa of 5.58 and 11.05), nicotinic acid (pKa values of 10.09, 9.14, 6.92, and 2.86), deoxymugineic acid ("DMA", pKa values of 9.55, 7.78, 3.40, and 2.72), diethylenetriaminepentaacetic acid ("DTPA", two pKa values greater than or equal to 6.30), 3,6,9-triaza-12-oxa-3,6,9-tricarboxymethylene-10-carboxy-13-phenyl-tridecanoic acid ("B-19036"), and combinations thereof. Other non-limiting examples of aminocarboxylic acids includes cyclic compounds such as 1,4,7,10-tetraazacyclododecane-N,N',N',N"'-tetraacetic acid ("DOTA"), p-isothiocyanatobenzyl-1,4,7,10-tetraazacyclododecane-1,4,7,10-tetraacetic acid ("p-SCN-Bz-DOTA"), 1,4,7,10-tetraazacyclododecane-N,N',N"-triacetic acid ("DO3A"), 1,4,7,10-tetraazacyclododecane-1,4,7,10-tetrakis(2-propionic acid) ("DOTMA"), 1,4,7-triazacyclononane-N,N',N"-triacetic acid ("NOTA"), 1,4,8,11-tetraazacyclotetradecane-N,N',N",N"'-tetraacetic acid ("TETA"), triethylenetetraaminehexaacetic acid ("TTHA"), trans-1,2-diaminohexanetetraacetic acid ("CYDTA"), 1,4,7,10-tetraazacyclododecane-1-(2-hydroxypropyl)4,7,10-triacetic acid ("HP-DO3A"), trans-cyclohexanediaminetetraacetic acid ("CDTA"), trans(1,2)-cyclohexanediethylenetriaminepentaacetic acid ("CDTPA"), 1-oxa-4,7,10-triazacyclododecane-N,N',N"-triacetic acid ("OTTA"), 1,4,7,10-tetraazacyclododecane-1,4,7,10-tetrakis {3-(4-carboxyl)-butanoic acid}, 1,4,7,10-tetraazacyclododecane-1,4,7,10-tetrakis(acetic acid-methyl amide), 1,4,7,10-tetraazacyclododecane-1,4,7,10-tetrakis(methylene phosphonic acid), and derivatives thereof, either individually or in combinations thereof. These aminopolycarboxylic acids are expected to have at least one pKa in the range from about 5 to about 10.

Non-limiting examples of iminocarboxylic acids include iminodiacetic acid (pKa of 2.98 and 9.89), 2-methoxyethyliminodiacetic acid (pKa of 2.2. and 8.96), 2-methylthioethyliminodiacetic acid (pKa of 2.1 and 8.91), N-2-sulfoethyliminodiacetic acid (pKa of 1.92, 2.28, and 8.16), N-(carbamoylmethyl)iminodiacetic acid (pKa of 2.30 and 6.60), and combinations thereof.

In an embodiment, the first of the two organic acids or salts thereof is EDTA or a salt thereof.

In another aspect, non-limiting examples of the second organic acid includes sorbic acid, acetic acid, dehydroacetic acid, proprionic acid, butyric acid, isobutyric acid, valeric acid, hexanoic acid (caproic acid), heptanoic acid (enanthic acid), octanoic acid (caprylic acid), nonanoic acid (pelargonic acid), decanoic acid (capric acid), (+) camphoric acid, peroxyacetic acid, n-peroxybutyric acid, peroxyformic acid, peroxypropionic acid, malonic acid, dimethylmalonic acid, succinic acid, glutaric acid, β-methylglutaric acid, adipic acid, pimelic acid, suberic acid, azelaic acid, 1,1-cyclopentanediacetic acid, 1,2-trans-cyclopentanedicarboxylic acid, 1,3-trans-cyclopentanedicarboxylic acid, 1,3-trans-cyclohexanedicarboxylic acid, 1,4-cis-cyclohexanedicarboxylic acid, cyclohexanecarboxylic acid, benzoic acid, methoxybenzoic acid, p-n-propoxybenzoic acid, p-n-butoxybenzoic acid, and combinations thereof. Their pKa values are shown in Table 1.

**Table 1**

| pKa Values of Some Organic Acids | |
|---|---|
| Acid Name | pKa |
| sorbic acid | 4.8 |
| acetic acid | 4.76 |
| dehydroacetic acid | 5.40 |
| propionic acid | 4.87 |
| butyric acid | 4.85 |
| isobutyric acid | 4.84 |
| valeric acid | 4.85 |
| hexanoic acid | 4.8 |
| heptanoic acid | 4.89 |
| octanoic acid | 4.89 |
| nonanoic acid | 4.95 |
| decanoic acid | 4.9 |
| (+) camphoric acid | 4.72 |
| peroxyacetic acid | 8.2 |
| n-peroxybutyric acid | 8.2 |
| peroxyformic acid | 7.1 |
| peroxypropionic acid | 8.1 |
| malonic acid | 2.83, 5.69 |
| dimethylmalonic acid | 3.17,6.06 |
| succinic acid | 4.19, 5.48 |
| glutaric acid | 4.34, 5.42 |
| β-methylglutaric acid | 4.25, 6.22 |
| adipic acid | 4.42, 5.41 |
| pimelic acid | 4.48, 5.42 |
| suberic acid | 4.52, 5.4 |
| azelaic acid | 4.55,5.41 |
| 1,1-cyclopentanediacetic acid | 3.82, 6.70 |
| 1,2-trans-cyclopentanedicarboxylic acid | 3.89,5.91 |
| 1,3-trans-cyclopentanedicarboxylic acid | 4.40, 5.45 |
| 1,3-trans-cyclohexanedicarboxylic acid | 4.18,5.93 |
| 1,4-cis-cyclohexanedicarboxylic acid | 4.44, 5.79 |
| cyclohexanecarboxylic acid | 4.90 |
| benzoic acid | 4.21 |
| p-methoxybenzoic acid | 4.47 |
| p-n-propoxybenzoic acid | 4.46 |
| p-n-butoxybenzoic acid | 4.53 |

In some embodiments, the second of said two organic acids is selected from the group consisting of sorbic acid, acetic acid, propionic acid, peroxyacetic acid, peroxypropionic acid, peroxyformic acid, cyclohexanecarboxylic acid, and combinations thereof.

In some other embodiments, the second of said two organic acids is selected from the group consisting of sorbic acid, acetic acid, dehydroacetic acid, propionic acid, peroxyacetic acid, peroxypropionic acid, and combinations thereof.

In still some other embodiments, the second of said two organic acids is selected from the group consisting of succinic acid, glutaric acid, β-methylglutaric acid, adipic acid, pimelic acid, suberic acid, azelaic acid, 1,1-cyclopentanediacetic acid, 1,2-trans-cyclopentanedicarboxylic acid, 1,3-trans-cyclopentanedicarboxylic acid, 1,3-trans-cyclohexanedicarboxylic acid, 1,4-cis-cyclohexanedicarboxylic acid, and combinations thereof.

In another aspect, said combination of at least two organic acids or salts thereof comprises three, four, five, or more organic acids or salts thereof.

In still another aspect, each of the two organic acids is present in a composition of the present invention at a concentration in a range from about 0.05 to about 0.5 percent by weight of the total composition. Alternatively, each of the two organic acids is present in a composition of the present invention at a concentration in a range from about 0.05 to about 0.3, or from about 0.1 to about 0.5, or from about 0.1 to about 0.3 percent by weight of the total composition.

The second of said two organic acids has a pKa that is no more than about 1.5 units less than the pH of the composition. Alternatively, said pKa is no more than about I unit less than the pH of the composition. In still another embodiment, said pKa is no more than about 0.5 unit less than the pH of the composition. In one embodiment, said second organic acid is a monocarboxylic acid.

The composition of the present invention further comprises a polyol.

Polyols suitable for use in a composition of the present invention include those having 2 to 18 (or, alternatively, 2 to 12, or 2 to 10, or 2 to 6, or 2 to 4) carbon atoms. In one embodiment, the polyol contains 2 to 6 carbon atoms. In another embodiment, the polyol contains 2 to 6 carbon atoms. Non-limiting examples of suitable polyols include glycerin, ethylene glycol, propylene glycol, sorbitol, mannitol, xylitol, monosaccharides, disaccharides, trisaccharides, and combinations thereof. In one embodiment, the polyol is selected from the group consisting of glycerin, ethylene glycol, propylene glycol, sorbitol, mannitol, xylitol, monosaccharides, and combinations thereof. In another embodiment, the polyol is selected from the group consisting of disaccharides. The polyol is a combination of glycerin and propylene glycol.

The concentration of a polyol included in a composition of the present invention is in a range from about 0.1 to about 1, or from about 0.1 to about 0.5, or from about 0.1 to about 0.3, or from about 0.2 to about 1 percent by weight of the total composition.

In another aspect, the ratio of alginate to polyol is in a range from about 1:20 to about 20:1. Alternatively, the ration is in a range from about 1:10 to about 10:1, or from about 1:7 to about 7:1, or from about 1:5 to about 5:1, or from about 1:3 to about 3:1.

In another embodiment, the alginate-containing composition is characterized in that it has a Mark-Houwink number that is a minimum of about 0.6. Typically, the Mark-Houwink number is desirably in a range from about 0.6 to about 1.2. In one embodiment, the Mark-Houwink number is about 1.

A composition is analyzed using size exclusion chromatography (SEC) with triple detection. Particularly, lights scattering, viscometry trace, and refractive index detection analysis are performed. The Mark-Houwink number is calculated from the data obtained from the triple detection SEC method using the mathematical technique disclosed in "Introduction to Physical Polymer Science," Third Edition, L. H. Sperling, Wiley-Interscience, John Wiley & Sons, Inc., New York, 2001. The shape of alginate particles in the composition may be inferred from the Mark-Houwink number as indicated in Table 2.

**Table 2**

| Values of the Mark-Houwink number | |
|---|---|
| Mark-Houwink number | Interpretation |
| 0 | spheres |
| 0.5 - 0.8 | random coils |
| 1.0 | stiff coils |
| 2.0 | rods |

In yet another aspect, a composition of the present invention is free of alexidine, chlorhexidine, parabens, benzalkonium chloride, polymeric quaternary ammonium compounds, and derivatives thereof.

The aqueous solutions employed in this invention may contain one or more additional ingredients that are commonly present in ophthalmic solutions, for example, tonicity-adjusting agents, buffers, antioxidants, viscosity-adjusting agents, surfactants, stabilizers, chelating agents, and the like, which aid in making ophthalmic compositions more comfortable to the user.

A composition of the present invention can be adjusted with tonicity-adjusting agents to approximate the tonicity of normal lacrimal fluids that is equivalent to a 0.9 percent (by weight) solution of sodium chloride or a 2.8 percent (by weight) of glycerin solution. The compositions of the present invention desirably have osmolality in a range from about 200 mOsm/kg to about 400 mOsm/ka. Alternatively, the osmolality is in the range from about 220 to about 360 mOsm/kg (or from about 220 to about 320 mOsm/kg, or from about 240 to about 300 mOsm/kg, or from about 240 to about 280 mOsm/kg, or from about 220 to about 280 mOsm/kg, or from about 220 to about 260 mOsm/kg).

The composition of the present invention comprises a buffering agent or system. Suitable buffers for use in compositions of the present invention include Good's buffers. Non-limiting examples of buffering agents include MES (2-(N-morpholino)ethanesulfonic acid hemisodium salt) having pKa of 6.1 at 25 °C and pH in the range of about 5.5-6.7; HEPES (N-{2-hydroxyethyl}peperazine-N'-{2-ethanesulfonic acid}) having pKₐ of 7.5 at 25 °C and pH in the range of about 6.8-8.2; BES (N,N-bis{2-hydroxyethyl}2-aminoethanesulfonic acid) having pKₐ of 7.1 at 25°C and pH in the range of about 6.4-7.8; MOPS (3-{N-morpholino}propanesulfonic acid) having pKₐ of 7.2 at 25°C and pH in the range of about 6.5-7.9; BIS-TRIS (bis(2-hydroxyethyl)amino-tris(hydroxymethyl)methane) having pKa of 6.5 at 25°C and pH in the range of about 5.8-7.2; citrate buffer (pH in the range of about 5.5-7.2); maleate buffer (pH in the range of about 5.5-7.2); succinate buffer (pH in the range of about 5.5-6.5); and malate buffer (pH in the range of about 4-6). Other pharmaceutically acceptable buffers that provide pH in the range of 5 to 7.5 also can be used.

A composition of the present invention can have a viscosity in the range from about 5 to about 100,000 centipoise ("cP") or mPa.s (or alternatively, from about 10 to about 50,000, or from about 10 to about 20,000, or from about 10 to about 10,000, or from about 10 to about 1,000, or from about 100 to about 10,000, or from about 100 to about 20,000, or from about 100 to about 50,000 or from about 500 to about 10,000, or from about 500 to about 20,000 cP or mPa.s).

The use of viscosity enhancing agents to provide the compositions of the invention with viscosities greater than the viscosity of simple aqueous solutions may be desirable to increase the retention time in the eye. Such viscosity enhancing agents include, for example, polyvinyl alcohol, polyvinyl pyrrolidone, methyl cellulose, hydroxypropylmethyl cellulose, hydroxyethyl cellulose, carboxymethyl cellulose, hydroxypropyl cellulose or other agents known to those skilled in the art. Such agents are typically employed at a level of from 0.01 to 10 percent (alternatively, 0.1 to 5 percent, or 0.1 to 2 percent) by weight.

Suitable surfactants include polyvinyl pyrrolidone, polyvinyl alcohol, polyethylene glycol, ethylene glycol, and propylene glycol. Other surfactants are polysorbates (such as polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of Tween^{®} 80, Tween^{®} 60, Tween^{®} 20), poloxamers (synthetic block polymers of ethylene oxide and propylene oxide, such as those commonly known by their trade names of Pluronic^{®}; e.g., Pluronic^{®} F127 or Pluronic^{®} F108)), or poloxamines (synthetic block polymers of ethylene oxide and propylene oxide attached to ethylene diamine, such as those commonly known by their trade names of Tetronic^{®}; e.g., Tetronic^{®} 1508 or Tetronic^{®} 908, etc., other nonionic surfactants such as Brij^{®}, Myrj^{®}, and long chain fatty alcohols (i.e., oleyl alcohol, stearyl alcohol, myristyl alcohol, docosohexanoyl alcohol, etc.) with carbon chains having about 12 or more carbon atoms (e.g., such as from about 12 to about 24 carbon atoms). A surfactant helps a topical formulation to spread on the ocular surface.

Suitable antioxidants include, but are not limited to, ascorbic acid and its esters, sodium bisulfite, butylated hydroxytoluene, butylated hydroxyanisole, tocopherols, and combinations thereof. Antioxidants can be included in a composition of the present invention in an amount in the range from about 0.005 to about 0.05 percent by weight (or alternatively, from about 0.005 to about 0.02 percent, or from about 0.005 to about 0.01 percent, by weight).

The present invention also provides a compound for use in ameliorating, reducing, treating, or preventing a condition of dry eye. The use comprises administering to an affected eye a composition that comprises: (a) alginate; (b) a combination of at least two organic acids or salts thereof; and (c) a pharmaceutically acceptable carrier; wherein the composition has a pH in a range from about 5 to about 7.5. In one embodiment, the composition has a pH in the range from about 5.5 to about 7.5. In another embodiment, the composition has a pH in the range from about 6 to about 7.5 (or alternatively, from about 6 to about 7, or from about 5.5 to about 7, or from about 5.5 to about 6.5).

In one aspect, the various ingredients of the composition are present in amounts disclosed herein.

In another aspect, the composition can be applied in one or more drops to an ocular surface once per day, twice per day, or three or more times per day, as needed.

In still another aspect, the use provides relief to an ocular discomfort resulting from a dry eye condition.

In still another aspect, at least one of said at least two organic acids or a salt thereof included in the composition has a pKa value in the range from about 6 to about 8.5.

In yet another aspect, each of said at least two organic acids or a salt thereof has a pKa value in the range from about 4 to about 10, and at least one organic acid or a salt thereof has a pKa value in the range from about 5 to about 10 (or alternatively, from about 5.5 to about 9, or from about 6 to about 9, or from about 6 to about 8.5). In one embodiment, at least one of the two organic acids has a pKa greater than the pH of the composition. In another embodiment, at least one of the two organic acids has a pKa that is at least one half unit greater than the pH of the composition. In still another embodiment, at least one of the two organic acids has a pKa that is at least 1 unit greater than the pH of the composition. It should be noted that a polycarboxylic acid has many pKa values, one or more of said pKa values need be greater than the pH of the composition. Preferably, a plurality of the pKa values of the polycarboxylic acid is greater than the pH of the composition. More preferably, a majority of the pKa values of the polycarboxylic acid is greater than the pH of the composition. Most preferably, all of the pKa values of the polycarboxylic acid are greater than the pH of the composition.

In a further aspect, the present invention provides a method for producing a composition for ameliorating, reducing, treating, or preventing a condition of dry eye. The method comprises combining: (1) alginate; (2) a combination of at least two organic acids or salts thereof; and (3) a pharmaceutically acceptable carrier, to form a mixture; wherein a pH of the mixture has a value in a range from about 5.5. to 6.5 to produce said composition.

The step of combining further includes adding a polyol into said mixture. Suitable polyols and their concentrations are disclosed herein above.

In yet another aspect, the method further comprises: (b) adjusting the pH value of the mixture to bring it into said pH range.

In a further aspect, the method further comprises: (c) subjecting the mixture to a sterilization procedure. In one embodiment, the sterilization procedure can comprise exposing the mixture to α, β, or y radiation; autoclaving the mixture; or heating the mixture to a temperature in arrange from about 100 to about 125 °C, for 10 minutes or longer, but less than a time that would result in a degradation of the alginate.

A composition of the present invention may be packaged in unit-dose (for single use) or multi-dose (for multiple use) containers.

Table 3 shows exemplary compositions that were prepared and tested.

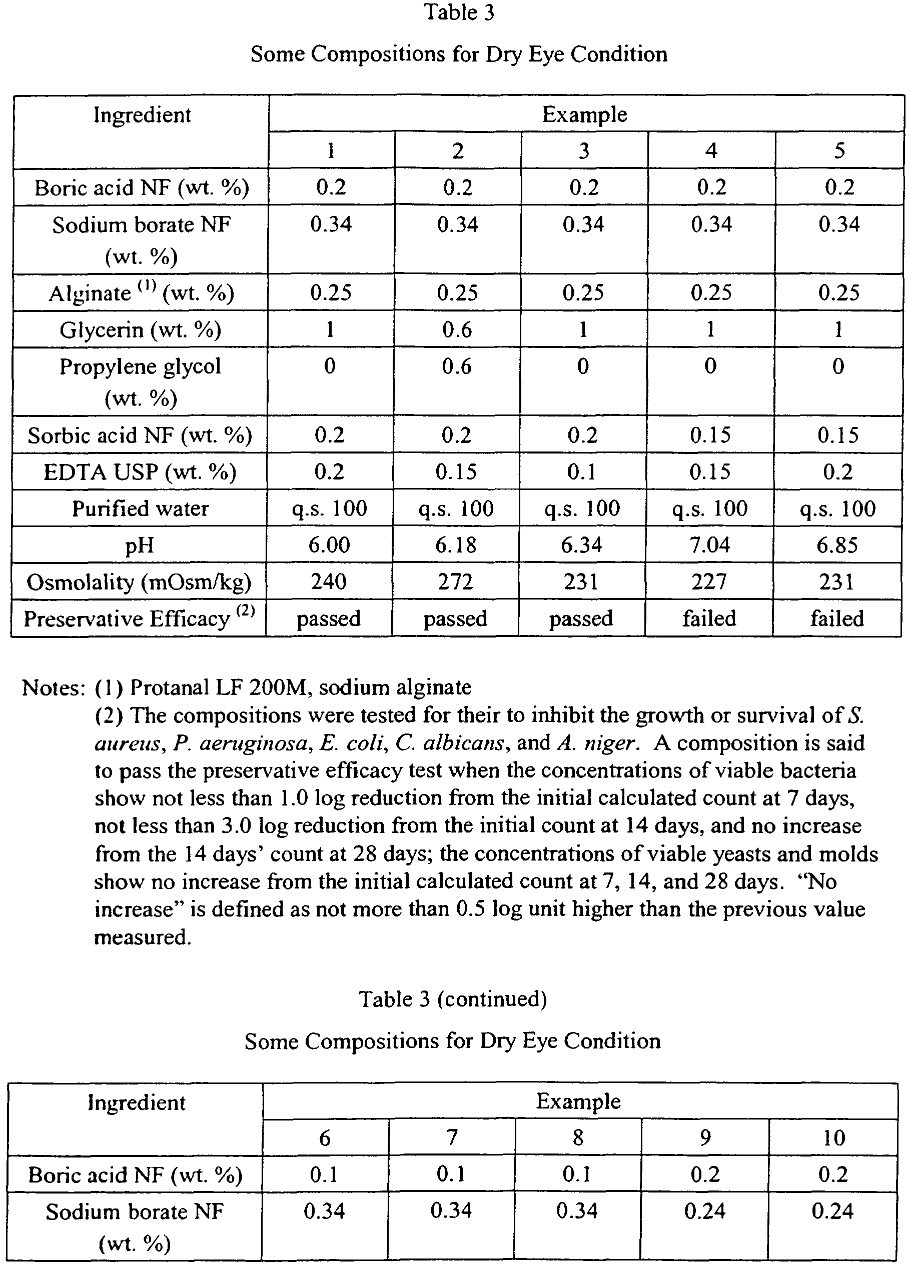

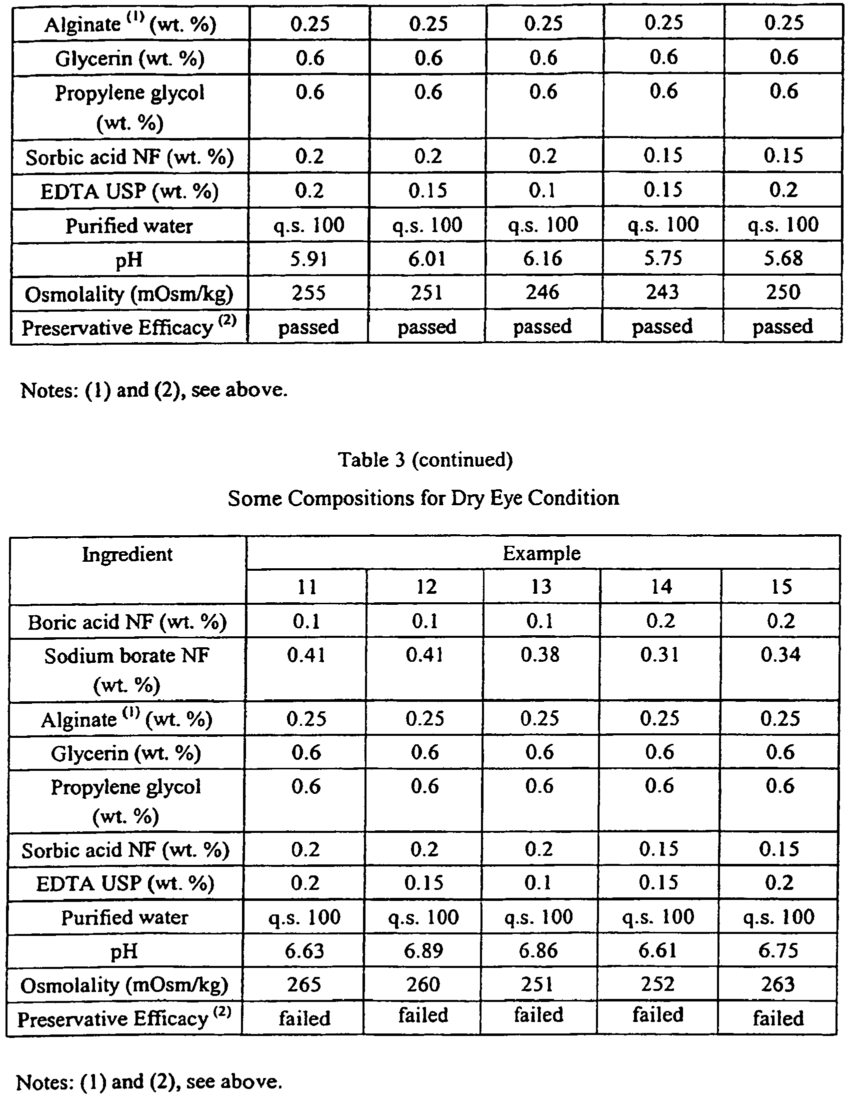

Examples 1, 3 to 5, and 11 to 15 are Reference Examples.

Although some compositions may fail in the long-term (28 days) preservative efficacy testing, they still may be sterilized and packaged in sterile containers for unit-dose use.

Table 4 shows some other exemplary compositions within the scope of the present invention that have not been experimentally prepared. These compositions are expected to have utility in providing relief to a dry eye condition. Table 4

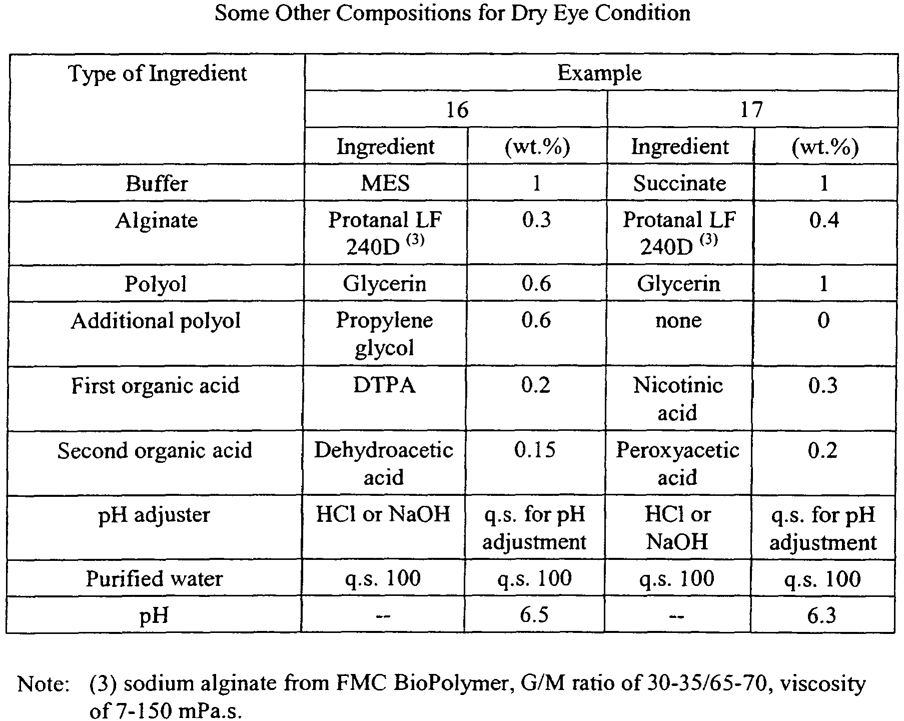

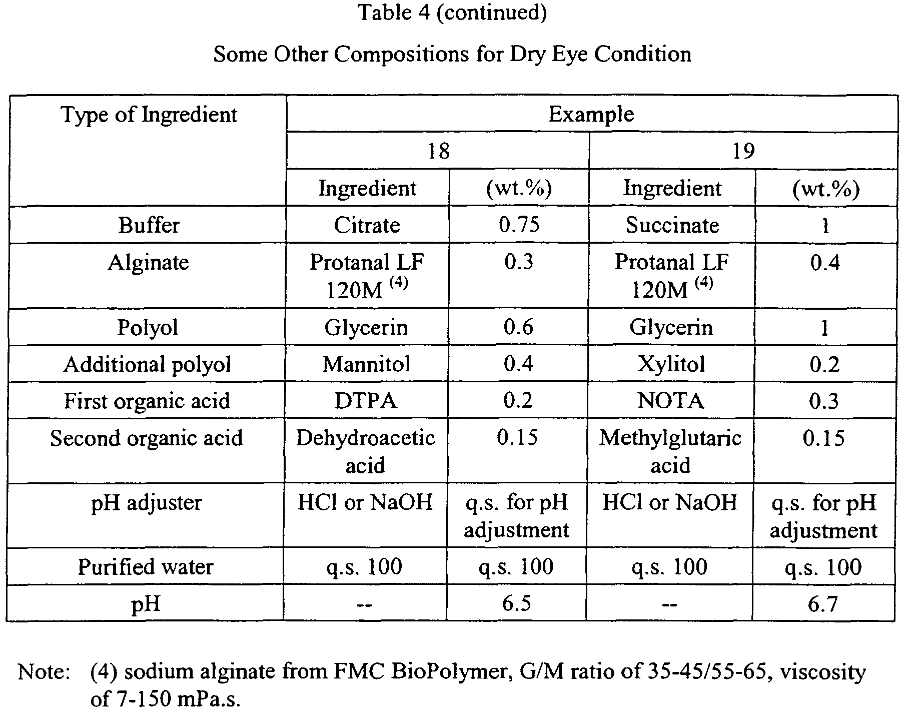

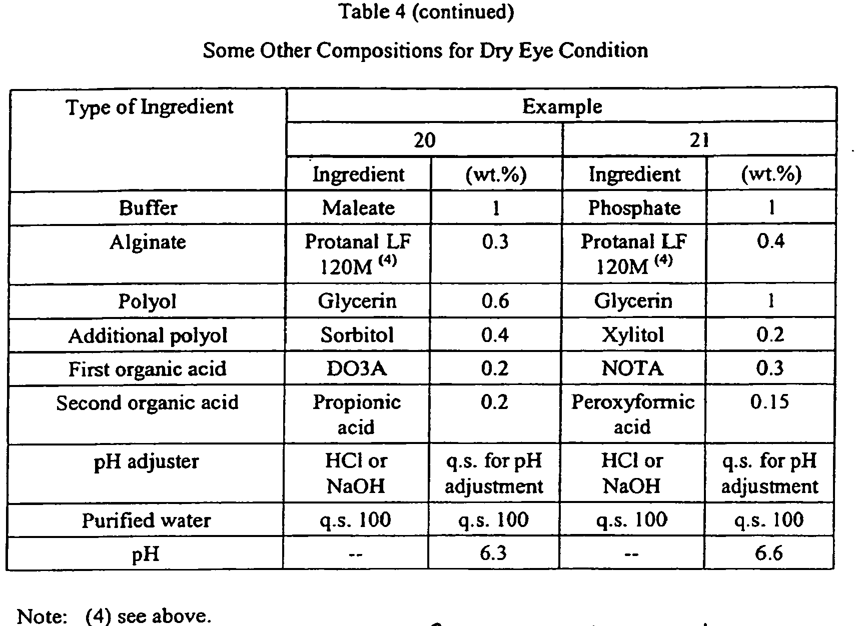

Examples 17 to 21 are Reference Examples.

The composition for reducing, ameliorating, treating, or preventing a condition of dry eye, the composition consists essentially of: (a) alginate in a concentration from about 0.1 to about 0.5 percent by weight of the total composition; (b) at least two organic acids or salts thereof, each present in a concentration from about 0.05 to about 0.5 percent by weight of the total composition; (c) glycerin in a concentration from about 0.1 to about 1 percent by weight of the total composition; (d) propylene glycol in a concentration from about 0.1 to about I percent by weight of the total composition; (e) a buffering system or agent; and (f) water; wherein the composition has a pH from about 5.5 to about 6.5; and at least one of said organic acids or salt thereof has a pKa value that is at least one half unit greater than said pH. The second organic acid has a pKa that is no more than 1.5 units less than the pH of the composition. In another embodiment, said buffering system or agent is boric acid/borate buffer.

In still another embodiment, a composition for reducing, ameliorating, treating, or preventing a condition of dry eye, the composition consists essentially of: (a) alginate in a concentration from about 0.1 to about 0.5 percent by weight of the total composition; (b) at least two organic acids or salts thereof, each present in a concentration from about 0.05 to about 0.5 percent by weight of the total composition; (c) glycerin in a concentration from about 0.1 to about 1 percent by weight of the total composition; (d) propylene glycol in a concentration from about 0.1 to about 1 percent by weight of the total composition; (e) a buffering system or agent; and (f) water; wherein the composition has a pH from about 5.5 to about 6.5; at least one of said organic acids or a salt thereof has a pKa value that is at least one half unit greater than said pH; and a second organic acid or salt thereof has a pKa value that is greater than about 4.5. In one embodiment, a second organic acid or salt thereof has a pKa that is no more than 1.5 units less than the pH of the composition. In another embodiment, said buffering system or agent is boric acid/borate buffer.

In yet another embodiment, a composition for reducing, ameliorating, treating, or preventing a condition of dry eye, the composition consists essentially of: (a) alginate in a concentration from about 0.1 to about 0.5 percent by weight of the total composition; (b) at least two organic acids or salts thereof, each present in a concentration from about 0.05 to about 0.5 percent by weight of the total composition; (c) glycerin in a concentration from about 0.1 to about 1 percent by weight of the total composition; (d) propylene glycol in a concentration from about 0.1 to about 1 percent by weight of the total composition; (e) a buffering system or agent; and (f) water; wherein the composition has a pH from about 5.5 to about 6.5; one of said organic acids or salt thereof is EDTA or a salt thereof; and a second organic acid or salt thereof is sorbic acid or a salt thereof. In one embodiment, a second organic acid has a pKa that is no more than 1.5 units less than the pH of the composition. In another embodiment, said buffering system or agent is boric acid/borate buffer.

In another aspect, any one of the compositions of the present invention can be formed into a solution, an emulsion (such as an oil-in-water emulsion), a dispersion, a gelable composition, or a gel.

In a typical production of a composition of the present invention, a volume of purified water that is equivalent to from about 85 to about 90 percent of the total batch weight (the temperature of purified water should be below 40°C before other ingredients are added) is added into a sterilized stainless steel mixing vessel equipped with a stirring mechanism. Alginate is added slowly with continued stirring and mixed thereafter for at least 30 minutes. Other ingredients are added slowly to the vessel over a period of about 30 minutes. The contents of the vessel is further mixed for another 15 minutes, then sterilized by any well-known method applicable for sterilization of pharmaceutical compositions. The composition is ready for packaging, storage, and use.

## Claims

1. An ophthalmic composition for use in reducing, ameliorating, treating, or preventing a condition of dry eye, the composition consists essentially of: (a) alginate in a concentration from 0.1 to 0.5 percent by weight of the total composition; (b) at least two organic acids or salts thereof, each present in a concentration from 0.05 to 0.5 percent by weight of the total composition; (c) glycerin in a concentration from 0.1 to 1 percent by weight of the total composition; (d) propylene glycol in a concentration from 0.1 to 1 percent by weight of the total composition; (e) a buffering system or agent; and (f) water; wherein the composition has a pH from 5.5 to 6.5; at least one of said organic acids or a salt thereof has a pKa value that is at least one half unit greater than said pH; and a second organic acid or a salt thereof has a pKa that is no more than 1.5 units less than said pH.

2. The ophthalmic composition of claim 1, wherein said at least one of said organic acids or a salt thereof is selected from the group consisting of ethylenediaminetetraacetic acid ("EDTA"), hexamethylenediaminetetraacetic acid ("HMDTA"), N-(2-hydroxyethyl)ethylenediamine-N,N',N'-triacetic acid ("HEEDTA" or HEDTA"), hydroxymethylethylenediaminetriacetic acid ("HMEDTA"), 1,3-diamino-2-propanol-N,N,N',N'-tetracetic acid, 1,3-diamino-2-propane-N,N,N',N'-tetracetic acid, ethylene glycol-bis(2-aminoethylether)-N,N,N',N'-tetraacetic acid, ethylenediamine-N,N-diacetic acid ("EDDA"), nicotinic acid, deoxymugineic acid ("DMA"), diethylenetriamine-pentaacetic acid ("DTPA"), 3,6,9-triaza-12-oxa-3,6,9-tricarboxymethylene-10-carboxy-13-phenyl-tridecanoic acid ("B-19036"), 1,4,7,10-tetraazacyclododecane-N,N',N",N"'-tetraacetic acid ("DOTA"), p-isothiocyanatobenzyl-1,4,7,10-tetraazacyclododecane-1,4,7,10-tetraacetic acid ("p-SCN-Bz-DOTA"), 1,4,7,10-tetraazacyclododecane-N,N',N"-triacetic acid ("DO3A"), 1,4,7,10-tetraazacyclododecane-1,4,7,10-tetrakis(2-propionic acid) ("DOTMA"), 1,4,7-triazacyclononane-N,N',N"-triacetic acid ("NOTA"), 1,4,8,11-tetraazacyclotetradecane-N,N',N",N"'-tetraacetic acid ("TETA"), triethylene tetraamine hexaacetic acid ("TTHA"), trans-1,2-diaminohexane tetraacetic acid ("CYDTA"), 1,4,7,10-tetraazacyclododecane-1-(2-hydroxypropyl)4,7,10-triacetic acid ("HP-DO3A"), trans-cyclohexanediaminetetraacetic acid ("CDTA"), trans(1,2)-cyclohexane diethylene triamine pentaacetic acid ("CDTPA"), 1-oxa-4,7,10-triazacyclododecane-N,N',N"-triacetic acid ("OTTA"), 1,4,7,10-tetraazacyclododecane-1,4,7,10-tetrakis {3-(4-carboxyl)-butanoic acid}, 1,4,7,10-tetraazacyclododecane-1,4,7,10-tetrakis(acetic acid methyl amide), 1,4,7,10-tetraazacyclododecane-1,4,7,10-tetrakis(methylene phosphonic acid), iminodiacetic acid, 2-methoxyethyliminodiacetic acid, 2-methylthioethyliminodiacetic acid, N-2-sulfoethyliminodiacetic acid, N-(carbamoylmethyl)iminodiacetic acid, salts thereof, and combinations thereof.

3. The ophthalmic composition of claim 2, wherein said at least one of said organic acids or a salt thereof is selected from the group consisting of EDTA, EDDA, DTPA, nicotinic acid, HEEDTA, DOTA, DO3A, OTTA, salts thereof, and combinations thereof.

4. The ophthalmic composition of claim 2, wherein said second organic acid or salt thereof is selected from the group consisting of sorbic acid, acetic acid, dehydroacetic acid, proprionic acid, butyric acid, isobutyric acid, valeric acid, hexanoic acid (caproic acid), heptanoic acid (enanthic acid), octanoic acid (caprylic acid), nonanoic acid (pelargonic acid), decanoic acid (capric acid), (+) camphoric acid, peroxyacetic acid, n-peroxybutyric acid, peroxyformic acid, peroxypropionic acid, malonic acid, dimethylmalonic acid, succinic acid, glutaric acid, β-methylglutaric acid, adipic acid, pimelic acid, suberic acid, azelaic acid, 1,1-cyclopentanediacetic acid, 1,2-trans-cyclopentanedicarboxylic acid, 1,3-trans-cyclopentanedicarboxylic acid, 1,3-trans-cyclohexanedicarboxylic acid, 1,4-cis-cyclohexanedicarboxylic acid, cyclohexanecarboxylic acid, benzoic acid, methoxybenzoic acid, p-n-propoxybenzoic acid, p-n-butoxybenzoic acid, salts thereof, and combinations thereof.

5. The ophthalmic composition of claim 4, wherein said second organic acid or salt thereof is selected from the group consisting of sorbic acid, acetic acid, dehydroacetic acid, propionic acid, peroxyacetic acid, peroxypropionic acid, salts thereof, and combinations thereof.

6. The ophthalmic composition of claim 4, wherein said second organic acid or salt thereof is selected from the group consisting of succinic acid, glutaric acid, β-methylglutaric acid, adipic acid, pimelic acid, suberic acid, azelaic acid, 1,1-cyclopentanediacetic acid, 1,2-trans-cyclopentanedicarboxylic acid, 1,3-trans-cyclopentanedicarboxylic acid, 1,3-trans-cyclohexanedicarboxylic acid, 1,4-cis-cyclohexanedicarboxylic acid, salts thereof, and combinations thereof.

7. The ophthalmic composition of claim 1, wherein said second organic acid has a pKa that is no more than 1 unit less than the pH of the composition.

8. The ophthalmic composition of claim 1, wherein one of said organic acid or salt thereof is EDTA or a salt thereof; and a second organic acid or salt thereof is sorbic acid or a salt thereof.

## Patentansprüche

1. Eine ophthalmische Zusammensetzung zur Verwendung bei der Reduzierung, Linderung, Behandlung oder Verhütung von trockenen Augen, die Zusammensetzung im wesentlichen bestehend aus: (a) Alginat in einer Konzentration von 0,1 bis 0,5 Gew.-%, bezogen auf das Gesamtgewicht der Zusammensetzung, (b) wenigstens zwei organische Säuren oder Salze davon, wobei jede in einer Konzentration von 0,05 bis 0,5 Gew.-%, bezogen auf das Gesamtgewicht der Zusammensetzung, vorhanden ist, (c) Glycerin in einer Konzentration von 0,1 bis 1 Gew.-%, bezogen auf das Gesamtgewicht der Zusammensetzung, (d) Propylenglycol in einer Konzentration von 0,1 bis 1 Gew.-%, bezogen auf das Gesamtgewicht der Zusammensetzung, (e) einem Puffersystem oder - agens und (f) Wasser, wobei die Zusammensetzung einen pH-Wert von 5,5 bis 6,5 aufweist, wenigstens eine der besagten organischen Säuren oder ein Salz davon einen pKa-Wert aufweist, der wenigstens eine halbe Einheit größer ist als besagter pH-Wert und eine zweite organische Säure oder ein Salz davon einen pKa-Wert aufweist, der nicht mehr als 1,5 Einheiten kleiner ist als besagter pH-Wert.

2. Die ophthalmische Zusammensetzung gemäß Anspruch 1, wobei wenigstens eine der besagten organischen Säuren oder das Salz davon ausgewählt ist aus der Gruppe bestehend aus Ethylendiamintetraessigsäure ("EDTA"), Hexamethylendiamintetraessigsäure ("HMDTA"), N-(2-Hydroxyethyl)ethylendiamin-N,N',N'-triessigsäure ("HEEDTA" oder HEDTA"), Hydroxymethylethylendiamintriessigsäure ("HMEDTA"), 1,3-Diamino-2-propanol-N,N,N',N'-tetraessigsäure, 1,3-Diamino-2-propan-N,N,N',N'-tetraessigsäure, Ethylenglycol-bis(2-aminoethylether)-N,N,N',N'-tetraessigsäure, Ethylenediamin-N,N-diessigsäure ("EDDA"), Nicotinsäure, Deoxymuginsäure ("DMA"), Diethylentriamin-pentaessigsäure ("DTPA"), 3,6,9-Triaza-12-oxa-3,6,9-tricarboxymethylen-10-carboxy-13-phenyl-tridecansäure ("B-19036"), 1,4,7,10-Tetraazacyclododecan-N,N',N",N"'-tetraessigsäure ("DOTA"), p-Isothiocyanatbenzyl-1,4,7,10-tetraazacyclododecan-1,4,7,10-tetraessigsäure ("p-SCN-Bz-DOTA"), 1,4,7,10-Tetraazacyclododecan-N,N',N"-triessigsäure ("DO3A"), 1,4,7,10-Tetraazacyclododecan-1,4,7,10-tetrakis(2-propionsäure) ("DOTMA"), 1,4,7-Triazacyclononan-N,N',N"-triessigsäure ("NOTA"), 1,4,8,11-Tetraazacyclotetradecan-N,N',N",N"'-tetraessigsäure ("TETA"), Triethylentetraaminhexaessigsäure ("TTHA"), trans-1,2-Diaminohexantetraessigsäure ("CYDTA"), 1,4,7,10-Tetraazacyclododecan-1-(2-hydroxypropyl)4,7,10-triessigsäure ("HP-DO3A"), trans-Cyclohexandiamintetraessigsäure ("CDTA"), trans(1,2)-Cyclohexandiethylentriaminpentaessigsäure ("CDTPA"), 1-Oxa-4,7,10-Triazacyclododecan-N,N',N"-triessigsäure ("OTTA"), 1,4,7,10-Tetraazacyclododecan-1,4,7,10-tetrakis{3-(4-carboxyl)-butansäure}, 1,4,7,10-Tetraazacydododecan-1,4,7,10-tetrakis(essigsäuremethylamid), 1,4,7,10-Tetraazacyclododecan-1,4,7,10-tetrakis(methylenphosphorsäure), Iminodiessigsäure, 2-Methoxyethyliminodiessigsäure, 2-Methylthioethyliminodiessigsäure, N-2-Sulfoethyliminodiessigsäure, N-(Carbamoylmethyl)iminodiessigsäure, Salzen davon und Kombinationen davon.

3. Die ophthalmische Zusammensetzung gemäß Anspruch 2, wobei wenigstens eine der besagten organischen Säuren oder das Salz davon ausgewählt ist aus der Gruppe bestehend aus EDTA, EDDA, DTPA, Nicotinsäure, HEEDTA, DOTA, DO3A, OTTA, Salzen davon und Kombinationen davon.

4. Die ophthalmische Zusammensetzung gemäß Anspruch 2, wobei besagte zweite organische Säure oder das Salz davon ausgewählt ist aus der Gruppe bestehend aus Sorbinsäure, Essigsäure, Dehydroessigsäure, Proprionsäure, Buttersäure, Isobuttersäure, Valeriansäure, Hexansäure (Capronsäure), Heptansäure (Önanthsäure), Octansäure (Caprylsäure), Nonansäure (Pelargonsäure), Decanonsäure (Caprinsäure), (+) Camphersäure, Peroxyessigsäure, n-Peroxybuttersäure, Peroxyameisensäure, Peroxypropionsäure, Malonsäure, Dimethylmalonsäure, Bernsteinsäure, Glutarsäure, β-Methylglutarsäure, Adipinsäure, Pimelinsäure, Suberinsäure, Azelainsäure, 1,1-Cyclopentandiessigsäure, 1,2-trans-Cyclopentandicarboxylsäure, 1,3-trans-Cyclopentandicarboxylsäure, 1,3-trans-Cyclohexandicarboxylsäure, 1,4-cis-Cyclohexandicarboxylsäure, Cyclohexancarboxylsäure, Benzoesäure, Methoxybenzoesäure, p-n-Propoxybenzoesäure, p-n-Butoxybenzoesäure, Salzen davon und Kombinationen davon.

5. Die ophthalmische Zusammensetzung gemäß Anspruch 4, wobei besagte zweite organische Säure oder das Salz davon ausgewählt ist aus der Gruppe bestehend aus Sorbinsäure, Essigsäure, Dehydroessigsäure, Propionsäure, Peroxyessigsäure, Peroxypropionsäure, Salzen davon und Kombinationen davon.

6. Die ophthalmische Zusammensetzung gemäß Anspruch 4, wobei besagte zweite organische Säure oder das Salz davon ausgewählt ist aus der Gruppe bestehend aus Succinsäure, Glutarsäure, β-Methylglutarsäure, Adipinsäure, Pimelinsäure, Suberinsäure, Azelainsäure, 1,1-Cyclopentandiessigsäure, 1,2-trans-Cyclopentandicarboxylsäure, 1,3-trans-Cyclopentandicarboxylsäure, 1,3-trans-Cyclohexandicarboxylsäure, 1,4-cis-Cyclohexandicarboxylsäure, Salzen davon und Kombinationen davon.

7. Die ophthalmische Zusammensetzung gemäß Anspruch 1, wobei besagte zweite organische Säure einen pKa-Wert aufweist, der nicht mehr als 1 Einheit kleiner ist als der pH-Wert der Zusammensetzung.

8. Die ophthalmische Zusammensetzung gemäß Anspruch 1, wobei eine der besagten organischen Säure oder des Salzes davon EDTA oder ein Salz davon ist und eine zweite organische Säure oder Salz davon Sorbinsäure oder ein Salz davon ist.

## Revendications

1. Composition ophtalmique pour utilisation dans la réduction, l'amélioration, le traitement ou la prévention d'un état de sécheresse oculaire, la composition étant constituée essentiellement de : (a) alginate à une concentration de 0,1 à 0,5 % en poids de la composition totale ; (b) au moins deux acides organiques ou sels de ceux-ci, chacun présent à une concentration de 0,05 à 0,5% en poids de la composition totale ; (c) glycérine à une concentration de 0,1 à 1 % en poids de la composition totale ; (d) propylèneglycol à une concentration de 0,1 à 1 % en poids de la composition totale ; (e) un système ou agent tampon ; et (f) eau ; laquelle composition a un pH de 5,5 à 6,5 ; dans laquelle au moins l'un desdits acides organiques ou sels de ceux-ci a une valeur pKa qui est supérieure d'au moins une demi-unité audit pH ; et un deuxième acide organique ou un sel de celui-ci a un pKa qui est inférieur d'au plus d'une unité et demie audit pH.

2. Composition ophtalmique selon la revendication 1, dans laquelle ledit au moins un desdits acides organiques ou de leurs sels est choisi dans le groupe constitué par l'acide éthylènediaminetétraacétique ("EDTA"), l'acide hexaméthylènediaminetétraacétique ("HMDTA"), l'acide N-(2-hydroxyéthyl)éthylènediamine-N,N',N'-triacétique ("HEEDTA" ou "HEDTA"), l'acide hydroxyméthyléthylènediaminetriacétique ("HMEDTA"), l'acide 1,3-diamino-2-propanol-N,N,N',N'-tétracétique, l'acide 1,3-diamino-2-propane-N,N,N',N'-tétracétique, l'acide éthylèneglycol-bis(2-aminoéthyléther)-N,N,N',N'-tétraacétique, l'acide éthylènediamine-N,N-diacétique ("EDDA"), l'acide nicotinique, l'acide désoxymuginéique ("DMA"), l'acide diéthylènetriaminepentaacétique ("DTPA"), l'acide 3,6,9-triaza-12-oxa-3,6,9-tricarboxyméthylène-10-carboxy-13-phényltridécanoïque ("B-19036"), l'acide 1,4,7,10-tétraazacyclododécane-N,N',N",N"'-tétraacétique ("DOTA"), l'acide p-isothiocyanatobenzyl-1,4,7,10-tétra-azacyclododécane-1,4,7,10-tétraacétique ("p-SCN-Bz-DOTA"), l'acide 1,4,7,10-tétraazacyclododécane-N,N',N"-triacétique ("DO3A"), l'acide 1,4,7,10-tétraazacyclododécane-1,4,7,10-tétrakis(2-propionique) ("DOTMA"), l'acide 1,4,7-triaza-cyclononane-N,N',N"-triacétique ("NOTA"), l'acide 1,4,8,11-tétraazacyclotétradécane-N,N',N",N"'-tétra-acétique ("TETA"), l'acide triéthylènetétramine-hexaacétique ("TTHA"), l'acide trans-1,2-diaminohexane-tétraacétique ("CYDTA"), l'acide 1,4,7,10-tétraazacyclo-dodécane-1-(2-hydroxypropyl)-4,7,10-triacétique ("HP-DO3A"), l'acide trans-cyclohexanediaminetétraacétique ("CDTA"), l'acide trans(1,2)-cyclohexanediéthylène-triaminepentaacétique ("CDTPA"), l'acide 1-oxa-4,7,10-triazacyclododécane-N,N',N"-triacétique ("OTTA"), l'acide 1,4,7,10-tétraazacyclododécane-1,4,7,10-tétrakis{3-(4-carboxyl)butanoïque}, le 1,4,7,10-tétraazacyclododécane-1,4,7,10-tétrakis(méthylamide d'acide acétique), l'acide 1,4,7,10-tétraazacyclododécane-1,4,7,10-tétrakis-(méthylènephosphonique), l'acide iminodiacétique, l'acide 2-méthoxyéthyliminodiacétique, l'acide 2-méthylthioéthyl-iminodiacétique, l'acide N-2-sulfoéthyliminodiacétique, l'acide N-(carbamoylméthyl)iminodiacétique, leurs sels, et leurs combinaisons.

3. Composition ophtalmique selon la revendication 2, dans laquelle ledit au moins un desdits acides organiques ou de leurs sels est choisi dans le groupe constitué par l'EDTA, l'EDDA, le DTPA, l'acide nicotinique, le HEEDTA, le DOTA, le DO3A, l'OTTA, leurs sels, et leurs combinaisons.

4. Composition ophtalmique selon la revendication 2, dans laquelle ledit deuxième acide organique ou son sel est choisi dans le groupe constitué par l'acide sorbique, l'acide acétique, l'acide déshydroacétique, l'acide propionique, l'acide butyrique, l'acide isobutyrique, l'acide valérique, l'acide hexanoïque (acide caproïque), l'acide heptanoïque (acide énanthique), l'acide octanoïque (acide caprylique), l'acide nonanoïque (acide pélargonique), l'acide décanoïque (acide caprique), l'acide (+)-camphorique, l'acide peroxyacétique, l'acide n-peroxybutyrique, l'acide peroxyformique, l'acide peroxypropionique, l'acide malonique, l'acide diméthylmalonique, l'acide succinique, l'acide glutarique, l'acide β-méthylglutarique, l'acide adipique, l'acide pimélique, l'acide subérique, l'acide azélaïque, l'acide 1,1-cyclopentanediacétique, l'acide 1,2-trans-cyclo-pentanedicarboxylique, l'acide 1,3-trans-cyclopentane-dicarboxylique, l'acide 1,3-trans-cyclohexanedicarboxylique, l'acide 1,4-cis-cyclohexanedicarboxylique, l'acide cyclohexanecarboxylique, l'acide benzoïque, l'acide méthoxybenzoïque, l'acide p-n-propoxybenzoïque, l'acide p-n-butoxybenzoïque, leurs sels, et leurs combinaisons.

5. Composition ophtalmique selon la revendication 4, dans laquelle ledit deuxième acide organique ou son sel est choisi dans le groupe constitué par l'acide sorbique, l'acide acétique, l'acide déshydroacétique, l'acide propionique, l'acide peroxyacétique, l'acide peroxypropionique, leurs sels, et leurs combinaisons.

6. Composition ophtalmique selon la revendication 4, dans laquelle ledit deuxième acide organique ou son sel est choisi dans le groupe constitué par l'acide succinique, l'acide glutarique, l'acide β-méthylglutarique, l'acide adipique, l'acide pimélique, l'acide subérique, l'acide azélaïque, l'acide 1,1-cyclopentanediacétique, l'acide 1,2-trans-cyclopentane-dicarboxylique, l'acide 1,3-trans-cyclopentane-dicarboxylique, l'acide 1,3-trans-cyclohexanedicarboxylique, l'acide 1,4-cis-cyclohexanedicarboxylique, leurs sels, et leurs combinaisons.

7. Composition ophtalmique selon la revendication 1, dans laquelle ledit deuxième acide organique a un pKa qui est inférieur d'au plus 1 unité au pH de la composition.

8. Composition ophtalmique selon la revendication 1, dans laquelle l'un desdits acides organiques ou sels de ceux-ci est l'EDTA ou un sel de celui-ci ; et un deuxième acide organique ou sel de celui-ci est l'acide sorbique ou un sel de celui-ci.
